# EUROPEAN PATENT SPECIFICATION

(11) **EP 4 041 086 B1**
(45) Date of publication and mention of the grant of the patent: **19.02.2025**
(21) Application number: 20786551.0
(22) Date of filing: 06.10.2020
(51) Int. Cl.: A61B 8/08, A61B 8/00

(54) **SYSTEMS AND METHODS FOR IMAGE OPTIMIZATION**
SYSTEME UND VERFAHREN ZUR BILDOPTIMIERUNG
SYSTÈMES ET PROCÉDÉS D'OPTIMISATION D'IMAGES

(30) Priority: 07.10.2019 US 201962911406 P
(43) Date of publication of application: 17.08.2022
(73) Proprietor: Koninklijke Philips N.V., 5656 AG Eindhoven (NL)
(72) Inventor: RAFTER, Patrick, Gabriels, 5656 AE Eindhoven (NL); SIVLEY, Roy, Allan, 5656 AE Eindhoven (NL); MATSUMURA, Towa, 5656 AE Eindhoven (NL); RADHAKRISHNAN, Kirthi, 5656 AE Eindhoven (NL); CANFIELD II, Earl, M., 5656 AE Eindhoven (NL); TRAHMS, Robert, Gustav, 5656 AE Eindhoven (NL)
(74) Representative: Philips Intellectual Property & Standards
(86) International application number: PCT/EP2020/078006
(87) International publication number: WO 2021/069445

(56) References cited:
- EP-A1- 3 469 993
- US-A1- 2010 260 398
- US-A1- 2018 136 896
- CHATELAIN PIERRE ET AL: "Confidence-driven control of an ultrasound probe: Target-specific acoustic window optimization", 2016 IEEE INTERNATIONAL CONFERENCE ON ROBOTICS AND AUTOMATION (ICRA), IEEE, 16 May 2016 (2016-05-16), pages 3441 - 3446, XP032908592, DOI: 10.1109/ICRA.2016.7487522

## Description

### TECHNICAL FIELD

The present disclosure pertains to imaging systems and methods for automated view recognition and image acquisition parameter adjustment. Particular implementations involve systems configured to automatically recognize an anatomical view and adjust acquisition parameters based on the anatomical view.

### BACKGROUND

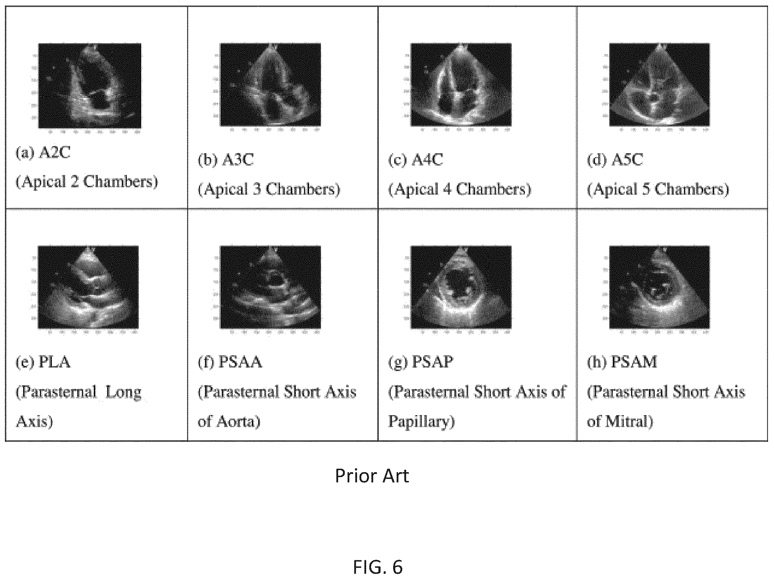

During an ultrasound exam, a sonographer scans a plane and/or volume in a subject to acquire one or more images. Typically, the sonographer acquires one or more standard views of the subject. A standard view is an image of anatomy from a particular location and angle that has been found to provide diagnostic value to a review (e.g., a radiologist). The number and types of standard views depend the type of ultrasound exam. For example, for an echocardiogram (e.g., an ultrasound exam including the heart) may include several standard views. Example ultrasound images of some of the standard views in an echocardiogram are illustrated in FIG. 6. The standard echocardiogram views may be used to assess the health of the heart. For example, the parasternal long axis (PLAX) view shown in panel (e) shows the left atrium, left ventricle, right ventricle, and mitral valve. The PLAX view may be used to diagnose certain cardiac conditions such as pericardial effusion (e.g., excess fluid surrounding the heart).

In addition to different locations and angles, different standard views may require different system parameters such as ultrasound image acquisition parameters and/or post-acquisition processing parameters, collectively referred to as imaging parameters. Imaging parameters include parameters such as lateral gain control, time gain compensation, transmission frequency, and power. Different imaging parameters may be needed due to the location (e.g., deep or shallow) and/or acoustic properties of the anatomy being scanned (e.g., heterogeneous, rigid) and/or properties of the acoustic window (e.g., between ribs) through which the standard view is acquired. For example, lateral gain control is used for apical views (Panes (a-d) of FIG. 6) to help visualize the walls of the heart but adds unwanted noise for parasternal views (Panes (e-h) of FIG. 6). In addition, time gain compensation (TGC) that is adjusted to better visualize the apex in apical windows results in over-compensation of the near-field for the parasternal long axis view.

There are also optimization trade-offs made based on views in color flow imaging, contrast imaging, xPlane, and 3D imaging. For example, in color flow, the primary direction of blood flow is towards and away from the transducer in an apical view but primarily perpendicular to the transducer in a parasternal view. In contrast imaging, apical windows often require a lower mechanical index than deeper parasternal views. The user has to manually adjust the power level to compensate for this increase in attenuation.

Individually optimizing all of the imaging parameters for each standard view is time consuming and often not practical in the time allotted for the ultrasound exam. Furthermore, many sonographers may not have enough expertise to fully optimize all of the imaging parameters for each standard view.

Currently, many commercial ultrasound systems provide "presets" which are sets of preprogrammed imaging parameters. A sonographer may select a type of exam (e.g., echocardiogram) and the ultrasound system may apply the preset for the echocardiogram. The preset allows the sonographer to acquire the standard views for the exam without having to adjust the imaging parameters. However, the imaging parameters of the preset are the result of trade-offs and compromises between the parameters. That is, while the imaging parameters of the preset may allow for adequate standard views to be acquired, none of the standard views may be acquired with imaging parameters optimized for that particular standard view. Accordingly, improving imaging parameters for standard views is desired.

US2010260398 discloses a system which provides volume imaging by implementing survey and target imaging modes. A survey imaging mode is implemented to provide a volume image of a relatively large survey area. A target of interest is identified within the survey area for use in a target imaging mode. A target imaging mode is implemented to provide a volume image of a relatively small target area corresponding to the identified target of interest. The target imaging mode preferably adapts the beamforming, volume field of view, and/or other signal and image processing algorithms to the target area.

### SUMMARY OF THE INVENTION

The present disclosure describes systems and methods for optimizing imaging parameters for specific views. Optimizing imaging parameters for specific views may allow for improved image quality without changing the workflow of users.

The invention is defined by the claims.

### SUMMARY OF THE DISCLOSURE

An ultrasound imaging system according to an example of the present disclosure may include an ultrasound transducer array configured to acquire an ultrasound image, a controller configured to control acquisition by the ultrasound transducer array based, at least in part, on one or more imaging parameters, a view recognition processor configured to determine if the ultrasound image corresponds to a specific view, and an optimization state controller configured to receive an output of the view recognition processor if the view recognition processor determines that the ultrasound image corresponds to the specific view, and to determine updates to the one or more imaging parameters, based, at least in part, on the output, wherein the optimization state controller provides the updated one or more imaging parameters to the controller.

A method according to an example of the present disclosure may include acquiring an ultrasound image, determining if the ultrasound image contains a specific view, if the specific view is determined, providing an output based on the specific view, determining one or more imaging parameters, based at least in part, on the output, providing the one or more imaging parameters to a controller, and reacquiring the ultrasound image with the one or more imaging parameters.

In accordance with an example of the present disclosure, a non-transitory computer-readable medium may contain instructions, that when executed, may cause an ultrasound imaging system to acquire an ultrasound image, determine if the ultrasound image contains a specific view, if the specific view is determined, provide an output based on the specific view, determine one or more imaging parameters, based at least in part, on the output, if the specific view is not determined, determining one or more default imaging parameters, provide the one or more imaging parameters or the one or more default imaging parameters to a controller, and reacquire the ultrasound image with the one or more imaging parameters or the one or more default imaging parameters to a controller.

### BRIEF DESCRIPTION OF THE DRAWINGS

FIG. **1** is a block diagram of an ultrasound system in accordance with principles of the present disclosure.
**FIG. 2** is a block diagram illustrating an example processor in accordance with principles of the present disclosure.
**FIG. 3** is a block diagram of a process for training and deployment of a neural network in accordance with the principles of the present disclosure.
**FIG. 4** is a flow chart of a method in accordance with principles of the present disclosure.
**FIG. 5** is a flow chart of a method in accordance with principles of the present disclosure.
**FIG. 6** shows example standard views for an echocardiography exam.
**FIGS. 7A and 7B** show example ultrasound images of a lateral heart wall.

### DETAILED DESCRIPTION

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. These embodiments are described in sufficient detail to enable those skilled in the art to practice presently disclosed systems and methods, and it is to be understood that other embodiments may be utilized and that structural and logical changes may be made without departing from and scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

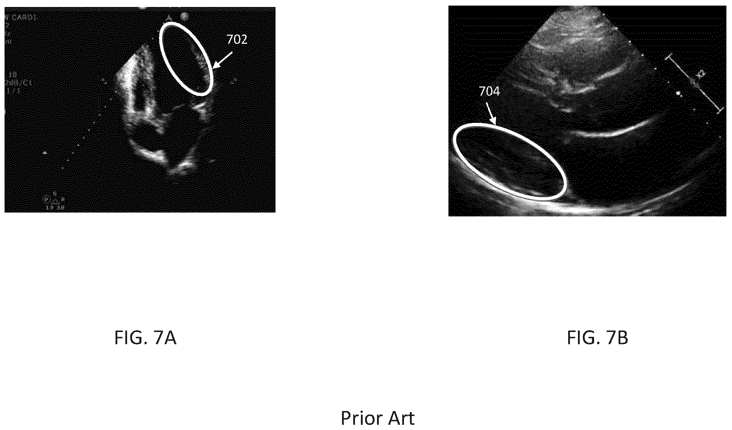

In cardiac ultrasound, there are several areas that are traditionally very difficult to image. In the apical 4 chamber view, the lateral wall is not well visualized. In this view the lateral wall is located on the edges of the sector and is indicated by circle 702 in FIG. 7A and circle 704 in FIG. 7B. In order to better visualize this wall of the heart the gains can be increased. The system has lateral gain controls to allow the user to control gain selectively at the edges of the image. However, when moving to the next view the user must reset these gains as the higher gains will introduce excessive noise. Often times due to the excessive noise, users will not optimize the lateral gains at all.

One emerging area of interest in echocardiography that may improve workflow is automated view classification. For example, a machine learning approach may be based on histogram analysis and statistical features. In another example, deep learning may be used to automate view recognition. Automated view recognition may improve workflow, specifically measurements and/or analysis as well as to aid in patient diagnosis. According to principles of the present disclosure, the view information provided by automated view recognition techniques may be used to adjust imaging parameters such as RF filters, time gain compensation (TGCs), lateral gain compensation (LGCs), and transmit frequency to improve image quality and workflow. Imaging parameters may include both acquisition parameters (e.g., settings for transmitting and receiving ultrasound signals) and post-acquisition parameters (e.g., settings for processing the received ultrasound signals). The principles may be applied to different imaging modes such as 2D echo imaging, color flow, contrast, xPlane, and 3D volume imaging.

For example, detecting the apical 4 chamber view and automatically adjusting the lateral gains may improve the visualization in the apical 4 view without making other views noisier. A further way to improve the visualization may be to provide different lateral gains for different parts of the cardiac cycle by automatically detecting the segment of the cardiac cycle in a given standard view. As the heart contracts the location of the lateral wall will change and it is not possible for the user of an ultrasound imaging system to compensate for this. However, compensation by the ultrasound imaging system may be achieved through detecting the location of the lateral wall through view recognition and changing the imaging parameters (e.g., changing the gain) throughout the cardiac cycle. Another compensation strategy that may be used is to change imaging parameters in a specific location (e.g., lower the receive RF filters where the lateral wall is located as detected by view recognition). The compensation by changing imaging parameters locally could be done in conjunction with a transmit frequency change on those acoustic lines. Lower frequencies will decrease attenuation and will improve signal to noise in the lateral wall. However, in other regions of the image that have already sufficient signal to noise the lower frequency will likely introduce undesired reverberation artifacts and lower the resolution, so the original frequency may be maintained in these regions.

According to principles of the present disclosure, view-specific optimization may be implemented by a view recognition processor, acquisition parameters that are optimized for imaging the views identified by the view recognition processor, and an optimization state controller that monitors outputs of the view recognition processor and applies the imaging parameters (e.g., view-specific system settings) in a manner that improves system responsiveness while reducing erratic transitions between imaging parameters that may be distracting to a user.

An ultrasound system in accordance with principles of the present invention may include or be operatively coupled to an ultrasound transducer array configured to transmit ultrasound signals toward a medium, e.g., a human body or specific portions thereof, and receive echoes responsive to the ultrasound signals. The ultrasound system may include a transmit controller and a beamformer configured to perform transmit and receive beamforming, respectively, and a display configured to display, in some embodiments, ultrasound images generated by the ultrasound imaging system.

The ultrasound imaging system may include one or more processors, such as a view recognition processor, which may include at least one model of a neural network in some embodiments. The neural network may be trained to determine whether a specific view (e.g., a standard view for a given exam type) has been acquired, and if so, which specific view. The view recognition processor may provide an output that includes an indication of the standard view acquired. In some applications, the indication of the standard view may include an indication of a physiological state, for example, a phase in the cardiac cycle in cardiac imaging. The indication of which standard view has been acquired may be provided to an optimization state controller. Based on the output of the view recognition processor (e.g., the indication of the standard view), the optimization state controller may retrieve an appropriate set of imaging parameters that may be optimized for the standard view determined by the view recognition processor. In some applications, the imaging parameters may be optimized not only for the specific view acquired but also for locations within the image, for example, a different gain setting may be used where the lateral wall of the heart is located. The imaging parameters may be provided to one or more elements of the ultrasound imaging system (e.g., the beamformer) to cause the ultrasound imaging system to acquire the specific view using the optimized imaging parameters. In some embodiments, the optimization state controller may only provide the imaging parameters when certain conditions are met. For example, when the ultrasound image acquired by the ultrasound imaging system has been stable for a certain period of time. This may prevent the user from being distracted by rapid changes in the imaging parameters.

The principles of the present disclosure may improve the quality of ultrasound images acquired for each specific view (e.g., less noise, improved visualization of anatomical structures, fewer artifacts).

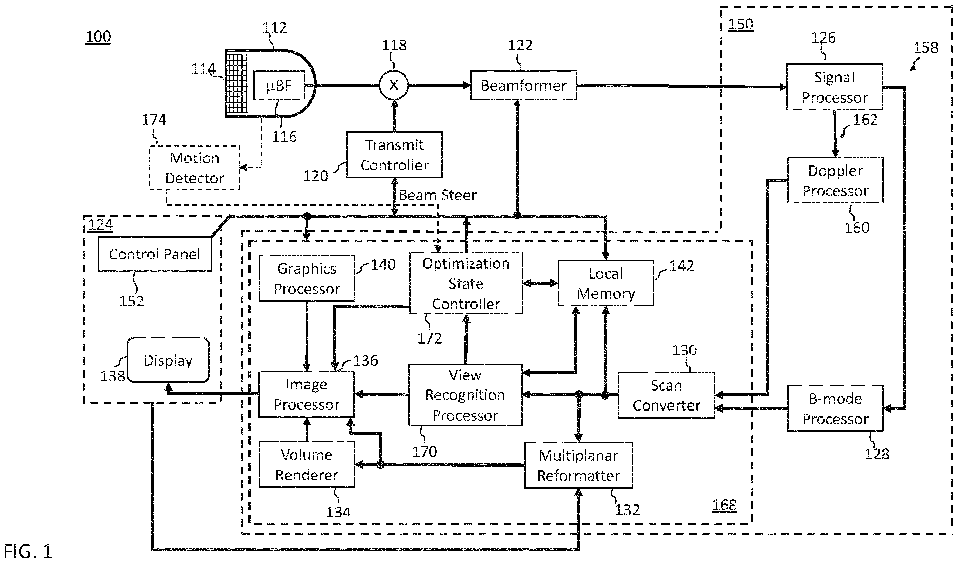

**FIG. 1** shows a block diagram of an ultrasound imaging system 100 constructed in accordance with the principles of the present disclosure. An ultrasound imaging system 100 according to the present disclosure may include a transducer array 114, which may be included in an ultrasound probe 112, for example an external probe or an internal probe such as an intravascular ultrasound (IVUS) catheter probe. In other embodiments, the transducer array 114 may be in the form of a flexible array configured to be conformally applied to a surface of subject to be imaged (e.g., patient). The transducer array 114 is configured to transmit ultrasound signals (e.g., beams, waves) and receive echoes responsive to the ultrasound signals. A variety of transducer arrays may be used, e.g., linear arrays, curved arrays, or phased arrays. The transducer array 114, for example, can include a two dimensional array (as shown) of transducer elements capable of scanning in both elevation and azimuth dimensions for 2D and/or 3D imaging. As is generally known, the axial direction is the direction normal to the face of the array (in the case of a curved array the axial directions fan out), the azimuthal direction is defined generally by the longitudinal dimension of the array, and the elevation direction is transverse to the azimuthal direction.

In some embodiments, the transducer array 114 may be coupled to a microbeamformer 116, which may be located in the ultrasound probe 112, and which may control the transmission and reception of signals by the transducer elements in the array 114. In some embodiments, the microbeamformer 116 may control the transmission and reception of signals by active elements in the array 114 (e.g., an active subset of elements of the array that define the active aperture at any given time).

In some embodiments, the microbeamformer 116 may be coupled, e.g., by a probe cable or wirelessly, to a transmit/receive (T/R) switch 118, which switches between transmission and reception and protects the main beamformer 122 from high energy transmit signals. In some embodiments, for example in portable ultrasound systems, the T/R switch 118 and other elements in the system can be included in the ultrasound probe 112 rather than in the ultrasound system base, which may house the image processing electronics. An ultrasound system base typically includes software and hardware components including circuitry for signal processing and image data generation as well as executable instructions for providing a user interface.

The transmission of ultrasonic signals from the transducer array 114 under control of the microbeamformer 116 is directed by the transmit controller 120, which may be coupled to the T/R switch 118 and a main beamformer 122. The transmit controller 120 may control the direction in which beams are steered. Beams may be steered straight ahead from (orthogonal to) the transducer array 114, or at different angles for a wider field of view. The transmit controller 120 may also be coupled to a user interface 124 and receive input from the user's operation of a user control. The user interface 124 may include one or more input devices such as a control panel 152, which may include one or more mechanical controls (e.g., buttons, encoders, etc.), touch sensitive controls (e.g., a trackpad, a touchscreen, or the like), and/or other known input devices.

In some embodiments, the partially beamformed signals produced by the microbeamformer 116 may be coupled to a main beamformer 122 where partially beamformed signals from individual patches of transducer elements may be combined into a fully beamformed signal. In some embodiments, microbeamformer 116 is omitted, and the transducer array 114 is under the control of the beamformer 122 and beamformer 122 performs all beamforming of signals. In embodiments with and without the microbeamformer 116, the beamformed signals of beamformer 122 are coupled to processing circuitry 150, which may include one or more processors (e.g., a signal processor 126, a B-mode processor 128, a Doppler processor 160, and one or more image generation and processing components 168) configured to produce an ultrasound image from the beamformed signals (i.e., beamformed RF data).

The signal processor 126 may be configured to process the received beamformed RF data in various ways, such as bandpass filtering, decimation, I and Q component separation, and harmonic signal separation. The signal processor 126 may also perform additional signal enhancement such as speckle reduction, signal compounding, and noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, Doppler image data). For example, the system may include a B-mode signal path 158 which couples the signals from the signal processor 126 to a B-mode processor 128 for producing B-mode image data.

The B-mode processor can employ amplitude detection for the imaging of structures in the body. The signals produced by the B-mode processor 128 may be coupled to a scan converter 130 and/or a multiplanar reformatter 132. The scan converter 130 may be configured to arrange the echo signals from the spatial relationship in which they were received to a desired image format. For instance, the scan converter 130 may arrange the echo signal into a two dimensional (2D) sector-shaped format, or a pyramidal or otherwise shaped three dimensional (3D) format. The multiplanar reformatter 132 can convert echoes which are received from points in a common plane in a volumetric region of the body into an ultrasonic image (e.g., a B-mode image) of that plane, for example as described in U.S. Pat. No. 6,443,896 (Detmer). The scan converter 130 and multiplanar reformatter 132 may be implemented as one or more processors in some embodiments.

A volume renderer 134 may generate an image (also referred to as a projection, render, or rendering) of the 3D dataset as viewed from a given reference point, e.g., as described in U.S. Pat. No. 6,530,885 (Entrekin et al.). The volume renderer 134 may be implemented as one or more processors in some embodiments. The volume renderer 134 may generate a render, such as a positive render or a negative render, by any known or future known technique such as surface rendering and maximum intensity rendering.

In some embodiments, the system may include a Doppler signal path 162 which couples the output from the signal processor 126 to a Doppler processor 160. The Doppler processor 160 may be configured to estimate the Doppler shift and generate Doppler image data. The Doppler image data may include color data which is then overlaid with B-mode (i.e. grayscale) image data for display. The Doppler processor 160 may be configured to filter out unwanted signals (i.e., noise or clutter associated with non-moving tissue), for example using a wall filter. The Doppler processor 160 may be further configured to estimate velocity and power in accordance with known techniques. For example, the Doppler processor may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some embodiments, the velocity and power estimates may undergo further threshold detection to further reduce noise, as well as segmentation and post-processing such as filling and smoothing. The velocity and power estimates may then be mapped to a desired range of display colors in accordance with a color map. The color data, also referred to as Doppler image data, may then be coupled to the scan converter 130, where the Doppler image data may be converted to the desired image format and overlaid on the B-mode image of the tissue structure to form a color Doppler or a power Doppler image.

According to principles of the present disclosure, output from the scan converter 130, such as B-mode images and Doppler images, referred to collectively as ultrasound images, may be provided to a view recognition processor 170. The view recognition processor 170 may analyze the ultrasound images to determine whether a specific view has been acquired. For example, if the imaging system is performing cardiac imaging, the view recognition processor 170 may be configured to determine whether a specific standard view of the heart (e.g., long or short axis parasternal, apical four-chamber view, or another standard view acquired via the subcostal/subxiphoid or apical windows) has been acquired. In some embodiments, the view recognition processor 170 may further determine a physiological state of the anatomy in the specific view. For instance and continuing with the cardiac imaging example, the physiological state may be a phase of a cardiac cycle for a standard view of the heart.

Based on the determination that the specific view has been acquired, the view recognition processor 170 may generate an output (e.g., signal). The output may include one or more signals or data that identify the specific view from the plurality of views analyzed by the processor 170 and/or the physiological state of the anatomy in the ultrasound image. In other examples, the output may include the image data that corresponds to the identified specific view and/or data representative of the physiological state of the anatomy. In some embodiments, the output may further include a signal or data that represents a confidence score. The confidence score may be a measure of the accuracy of the view identification by the view recognition processor 170. That is, the confidence score may represent a likelihood or probability that the view identified as the specific view by the processor 170 does in fact correspond to the desired specific view and/or physiological state.

In some embodiments, the view recognition processor 170 may utilize a neural network, for example a deep neural network (DNN), a convolutional neural network (CNN), a recurrent neural network (RNN), an autoencoder neural network, or the like, to recognize specific views. The neural network may be implemented in hardware (e.g., neurons are represented by physical components) and/or software (e.g., neurons and pathways implemented in a software application) components. The neural network implemented according to the present disclosure may use a variety of topologies and learning algorithms for training the neural network to produce the desired output. For example, a software-based neural network may be implemented using a processor (e.g., single or multi-core CPU, a single GPU or GPU cluster, or multiple processors arranged for parallel-processing) configured to execute instructions, which may be stored in computer readable medium, and which when executed cause the processor to perform a trained algorithm for determining whether a specific view has been acquired.

In various embodiments, the neural network(s) may be trained using any of a variety of currently known or later developed learning techniques to obtain a neural network (e.g., a trained algorithm or hardware-based system of nodes) that is configured to analyze input data in the form of ultrasound images, measurements, and/or statistics and determine whether a specific view has been acquired and which specific view has been acquired. In some embodiments, the neural network may be statically trained. That is, the neural network may be trained with a data set and deployed on the view recognition processor 170. In some embodiments, the neural network may be dynamically trained. In these embodiments, the neural network may be trained with an initial data set and deployed on the view recognition processor 170. However, the neural network may continue to train and be modified based on ultrasound images acquired by the system 100 after deployment of the neural network on the view recognition processor 170.

In other embodiments, the view recognition processor 170 may not include a neural network. In other embodiments, the view recognition processor 170 may be implemented using any other suitable image processing technique, such as image segmentation, histogram analysis, edge detection or other shape or object recognition techniques. In some embodiments, the view recognition processor 170 may implement a neural network in combination with other image processing methods to recognize specific views.

Although reference is made to indications of standard views (e.g., the views expected for a particular exam type for making a diagnosis or other assessment), in some embodiments, the neural network may be trained to recognize and provide an indication of any view desired by the user. For example, in clinical studies where the utility of non-standard views is being evaluated or for novel indications where no standard views have yet been established (e.g., monitoring of new diseases, imaging novel implanted medical devices).

In some embodiments, the view recognition processor 170 may provide its output to an optimization state controller 172. The optimization state controller 172 may be implemented in any suitable hardware and/or software. In some embodiments, the optimization state controller 172 may be implemented by one or more processors, which responsive to the output of the view recognition processor 170, determines appropriate imaging parameters for the specific view. Imaging parameters determined by the optimization state controller 172 may include, but are not limited to, RF filters, TGCs, LGCs, and transmit frequency. In some embodiments, determining the appropriate imaging parameters may include referencing a lookup table stored in memory (e.g., local memory 142) and retrieving the appropriate acquisition parameters for the specific view from the memory (e.g., local memory 142). In some such examples, the look up table may be implemented using any suitable relational data structure, which relates a specific view (e.g., a standard apical four-chamber view) to a specific set of imaging parameters (e.g., specific TGCs, LGCs, and transmit frequency settings).

In some embodiments, one or more of the imaging parameters may be uniform across the scan area of the ultrasound image. In other embodiments, one or more of the imaging parameters may vary across the scan area of the ultrasound image. For example, one or more imaging parameters may be different where an anatomical feature is located in the specific view. Continuing with the echocardiography example, if a lateral wall of the heart is located in the specific view, the imaging parameters may be adjusted in the scan area where the lateral wall of the heart is located. For example, the transmit frequency may be reduced in the area of the lateral wall to improve visualization of the lateral wall, but the transmit frequency may be higher in other portions of the scan area to reduce introduction of excessive noise. In other examples, where acoustic properties of tissue may be more homogenous, such as hepatic imaging, the gain or other imaging parameters may be uniform across the scan area.

Some or all of the imaging parameters determined by the optimization state controller 172 may be provided to the transmit controller 120 and/or beamformer 122. The transmit controller 120 and/or beamformer 122 may cause the ultrasound image to be acquired with the determined imaging parameters (e.g., view-specific imaging parameters). Some or all of the determined imaging parameters may also or alternatively be provided to an image processor 136. The image processor 136 may process the acquired ultrasound image based on the imaging parameters and provide the processed ultrasound image to the display 138.

The optimization state controller 172 is responsible for controlling the imaging parameters of the ultrasound imaging system 100 over time. The optimization state controller 172 may maintain the current imaging parameters, monitoring the outputs of the view recognition processor 170, and combing this information to determine if and when the imaging parameters should be changed. When the optimization state controller 172 triggers an imaging parameter change, it may replace its record of the current imaging parameters with the newly chosen imaging parameters, provides the new imaging parameters to other components of the system 100 as described above, and then resumes monitoring the view recognition processor 170 outputs for potential future imaging parameter changes.

The optimization state controller 172 may provide the user with an optimal balance of system responsiveness and stability. If the optimization state controller 172 responds too quickly to certain view recognition processor 170 outputs, the incorrect imaging parameters could be chosen and/or the system 100 could change imaging parameters so quickly that the display 138 becomes erratic and the image unusable. In either case, the user may lose confidence in the ability of the system 100 to provide reliable diagnostic imaging. Thus, in some embodiments, the optimization state controller 172 may wait for one or more conditions prior to determining or providing determined imaging parameters. For example, the optimization state controller 172 may wait until the indication provided by the view recognition processor 170 is stable for a certain period of time (e.g., 0.5s, 1s, 2s) or a certain number of image frames (e.g., 5, 10, 30). In some embodiments, the optimization state controller 172 may analyze confidence scores provided by the view recognition processor 170, possibly over multiple image frames, and determine if and when the view recognition processor 170 is sufficiently confident prior to determining or providing the imaging parameters, for example, when the confidence scores are above a threshold value (e.g., 70%, 90%) for one or more frames. In some embodiments, the threshold value for the confidence score may be preset. In other embodiments, the threshold value may be set by a user input.

Optionally, in some embodiments, the ultrasound probe 112 may include or be coupled to a motion detector 174. The motion detector 174 may provide a signal to the optimization state controller 172 to indicate when the ultrasound probe 112 is moving and when it is stationary. In some embodiments, the optimization state controller 172 may wait for the signal to indicate the ultrasound probe 112 is stationary prior to determining or providing determined imaging parameters. In some embodiments, the optimization state controller 172 may wait for the signal to indicate the ultrasound probe 112 is stationary for a set period of time (e.g., 0.5 sec, 1 sec, 2 sec) prior to determining or providing determined imaging parameters.

In some embodiments, when the indication of the specific view is unstable and/or the confidence score is below the threshold, for example, when the user is actively moving the transducer to find a suitable acoustic window, the optimization state controller 172 may maintain the previous imaging parameters or provide default, non-view-specific imaging parameters until confidence in the recognized view can be established. In some embodiments, the default imaging parameters may be based on exam type or other presets.

Output (e.g., B-mode images, Doppler images) from the scan converter 130, the multiplanar reformatter 132, and/or the volume renderer 134 may be coupled to an image processor 136 for further enhancement, buffering and temporary storage before being displayed on an image display 138. Although output from the scan converter 130 is shown as provided to the image processor 136 via the view recognition processor 170, in some embodiments, the output of the scan converter 130 may be provided directly to the image processor 136. A graphics processor 140 may generate graphic overlays for display with the images. These graphic overlays can contain, e.g., standard identifying information such as patient name, date and time of the image, imaging parameters, and the like. For these purposes the graphics processor may be configured to receive input from the user interface 124, such as a typed patient name or other annotations. The user interface 144 can also be coupled to the multiplanar reformatter 132 for selection and control of a display of multiple multiplanar reformatted (MPR) images.

The system 100 may include local memory 142. Local memory 142 may be implemented as any suitable non-transitory computer readable medium (e.g., flash drive, disk drive). Local memory 142 may store data generated by the system 100 including ultrasound images, executable instructions, imaging parameters, training data sets, or any other information necessary for the operation of the system 100.

As mentioned previously system 100 includes user interface 124. User interface 124 may include display 138 and control panel 152. The display 138 may include a display device implemented using a variety of known display technologies, such as LCD, LED, OLED, or plasma display technology. In some embodiments, display 138 may comprise multiple displays. The control panel 152 may be configured to receive user inputs (e.g., exam type, threshold confidence score). The control panel 152 may include one or more hard controls (e.g., buttons, knobs, dials, encoders, mouse, trackball or others). In some embodiments, the control panel 152 may additionally or alternatively include soft controls (e.g., GUI control elements or simply, GUI controls) provided on a touch sensitive display. In some embodiments, display 138 may be a touch sensitive display that includes one or more soft controls of the control panel 152.

In some embodiments, various components shown in FIG. 1 may be combined. For instance, image processor 136 and graphics processor 140 may be implemented as a single processor. In another example, the scan converter 130 and multiplanar reformatter 132 may be implemented as a single processor. In some embodiments, various components shown in FIG. 1 may be implemented as separate components. For example, signal processor 126 may be implemented as separate signal processors for each imaging mode (e.g., B-mode, Doppler). In some embodiments, one or more of the various processors shown in FIG. 1 may be implemented by general purpose processors and/or microprocessors configured to perform the specified tasks. In some embodiments, one or more of the various processors may be implemented as application specific circuits. In some embodiments, one or more of the various processors (e.g., image processor 136) may be implemented with one or more graphical processing units (GPU).

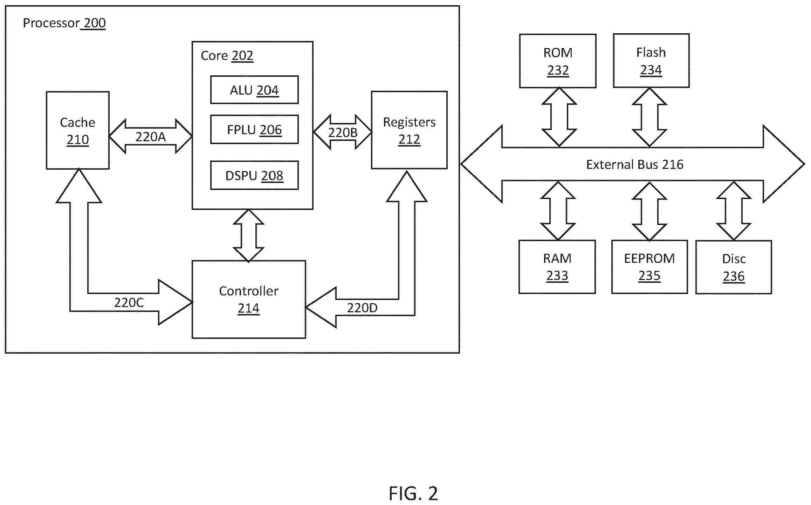

**FIG. 2** is a block diagram illustrating an example processor 200 according to principles of the present disclosure. Processor 200 may be used to implement one or more processors and/or controllers described herein, for example, image processor 136 shown in FIG. 1 and/or any other processor or controller shown in FIG. 1. Processor 200 may be any suitable processor type including, but not limited to, a microprocessor, a microcontroller, a digital signal processor (DSP), a field programmable gate array (FPGA) where the FPGA has been programmed to form a processor, a graphical processing unit (GPU), an application specific circuit (ASIC) where the ASIC has been designed to form a processor, or a combination thereof.

The processor 200 may include one or more cores 202. The core 202 may include one or more arithmetic logic units (ALU) 204. In some embodiments, the core 202 may include a floating point logic unit (FPLU) 206 and/or a digital signal processing unit (DSPU) 208 in addition to or instead of the ALU 204.

The processor 200 may include one or more registers 212 communicatively coupled to the core 202. The registers 212 may be implemented using dedicated logic gate circuits (e.g., flip-flops) and/or any memory technology. In some embodiments the registers 212 may be implemented using static memory. The register may provide data, instructions and addresses to the core 202.

In some embodiments, processor 200 may include one or more levels of cache memory 210 communicatively coupled to the core 202. The cache memory 210 may provide computer-readable instructions to the core 202 for execution. The cache memory 210 may provide data for processing by the core 202. In some embodiments, the computer-readable instructions may have been provided to the cache memory 210 by a local memory, for example, local memory attached to the external bus 216. The cache memory 210 may be implemented with any suitable cache memory type, for example, metal-oxide semiconductor (MOS) memory such as static random access memory (SRAM), dynamic random access memory (DRAM), and/or any other suitable memory technology.

The processor 200 may include a controller 214, which may control input to the processor 200 from other processors and/or components included in a system (e.g., control panel 152 and scan converter 130 shown in FIG. 1) and/or outputs from the processor 200 to other processors and/or components included in the system (e.g., display 138 and volume renderer 134 shown in FIG. 1). Controller 214 may control the data paths in the ALU 204, FPLU 206 and/or DSPU 208. Controller 214 may be implemented as one or more state machines, data paths and/or dedicated control logic. The gates of controller 214 may be implemented as standalone gates, FPGA, ASIC or any other suitable technology.

The registers 212 and the cache 210 may communicate with controller 214 and core 202 via internal connections 220A, 220B, 220C and 220D. Internal connections may implemented as a bus, multiplexor, crossbar switch, and/or any other suitable connection technology.

Inputs and outputs for the processor 200 may be provided via a bus 216, which may include one or more conductive lines. The bus 216 may be communicatively coupled to one or more components of processor 200, for example the controller 214, cache 210, and/or register 212. The bus 216 may be coupled to one or more components of the system, such as display 138 and control panel 152 mentioned previously.

The bus 216 may be coupled to one or more external memories. The external memories may include Read Only Memory (ROM) 232. ROM 232 may be a masked ROM, Electronically Programmable Read Only Memory (EPROM) or any other suitable technology. The external memory may include Random Access Memory (RAM) 233. RAM 233 may be a static RAM, battery backed up static RAM, Dynamic RAM (DRAM) or any other suitable technology. The external memory may include Electrically Erasable Programmable Read Only Memory (EEPROM) 235. The external memory may include Flash memory 234. The external memory may include a magnetic storage device such as disc 236. In some embodiments, the external memories may be included in a system, such as ultrasound imaging system 100 shown in Fig. 1, for example local memory 142.

In some embodiments, the system 100 can be configured to implement a neural network included in the view recognition processor 170, which may include a CNN, to determine whether a specific view has been acquired, which specific view has been acquired, a physiological state of the specific view, and/or a confidence score. The neural network may be trained with imaging data such as image frames where one or more items of interest are labeled as present. Neural network may be trained to recognize target anatomical features associated with specific ultrasound exams (e.g., different standard views of the heart for echocardiography) or a user may train neural network to locate one or more custom target anatomical features (e.g., implanted device, liver tumor).

In some embodiments, a neural network training algorithm associated with the neural network can be presented with thousands or even millions of training data sets in order to train the neural network to determine a confidence level for each measurement acquired from a particular ultrasound image. In various embodiments, the number of ultrasound images used to train the neural network(s) may range from about 50,000 to 200,000 or more. The number of images used to train the network(s) may be increased if higher numbers of different items of interest are to be identified, or to accommodate a greater variety of patient variation, e.g., weight, height, age, etc. The number of training images may differ for different items of interest or features thereof, and may depend on variability in the appearance of certain features. For example, tumors typically have a greater range of variability than normal anatomy. Training the network(s) to assess the presence of items of interest associated with features for which population-wide variability is high may necessitate a greater volume of training images.

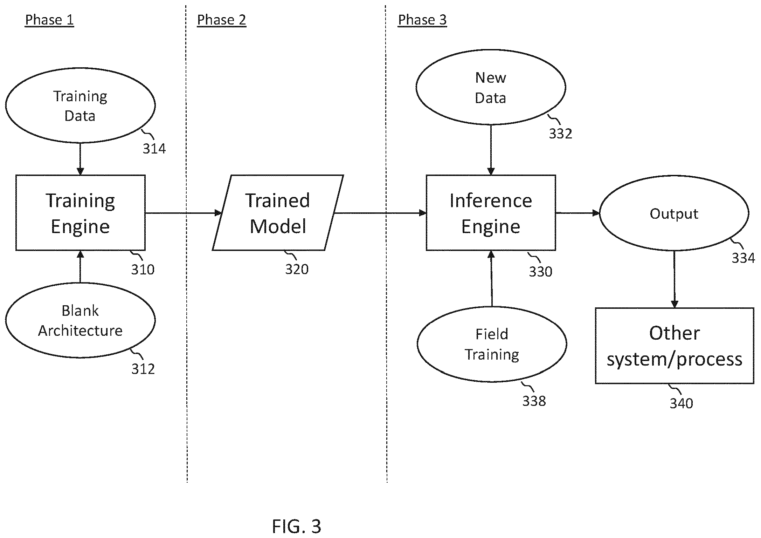

FIG. **3** shows a block diagram of a process for training and deployment of a neural network in accordance with the principles of the present disclosure. The process shown in FIG. 3 may be used to train a neural network included in view recognition processor 170. The left hand side of FIG. 3, phase 1, illustrates the training of a neural network. To train the neural network, training sets which include multiple instances of input arrays and output classifications may be presented to the training algorithm(s) of the neural network(s) (e.g., AlexNet training algorithm, as described by Krizhevsky, A., Sutskever, I. and Hinton, G. E. "ImageNet Classification with Deep Convolutional Neural Networks," NIPS 2012 or its descendants). Training may involve the selection of a starting network architecture 312 and the preparation of training data 314. The starting network architecture 312 may be a blank architecture (e.g., an architecture with defined layers and arrangement of nodes but without any previously trained weights) or a partially trained network, such as the inception networks, which may then be further tailored for classification of ultrasound images. The starting architecture 312 (e.g., blank weights) and training data 314 are provided to a training engine 310 for training the model. Upon sufficient number of iterations (e.g., when the model performs consistently within an acceptable error), the model 320 is said to be trained and ready for deployment, which is illustrated in the middle of FIG. **3****,** phase 2. The right hand side of FIG. **3****,** or phase 3, the trained model 320 is applied (via inference engine 330) for analysis of new data 332, which is data that has not been presented to the model during the initial training (in phase 1). For example, the new data 332 may include unknown images such as live ultrasound images acquired during a scan of a patient (e.g., cardiac images during an echocardiography exam). The trained model 320 implemented via engine 330 is used to classify the unknown images in accordance with the training of the model 320 to provide an output 334 (e.g., specific view, physiological state, confidence score). The output 334 may then be used by the system for subsequent processes 340 (e.g., as input to the optimization state controller 172 for determining the imaging parameters).

In embodiments where the neural network is dynamically trained, the engine 330 may receive field training 338. The engine 330 may continue to train and be modified based on data acquired after deployment of the engine 330. The field training 338 may be based, at least in part, on new data 332 in some embodiments.

In the embodiments where the trained model 320 is used to implement a neural network of the view recognition processor 170, the starting architecture may be that of a convolutional neural network, or a deep convolutional neural network, which may be trained to perform image frame indexing, image segmentation, image comparison, or any combinations thereof. With the increasing volume of stored medical image data, the availability of high-quality clinical images is increasing, which may be leveraged to train a neural network to learn the probability of a given image frame containing a given specific view (e.g., confidence score). The training data 314 may include multiple (hundreds, often thousands or even more) annotated/labeled images, also referred to as training images. It will be understood that the training image need not include a full image produced by an imagining system (e.g., representative of the full field of view of the probe) but may include patches or portions of images of the labeled item of interest.

In various embodiments, the trained neural network may be implemented, at least in part, in a computer-readable medium comprising executable instructions executed by a processor, e.g., view recognition processor 170.

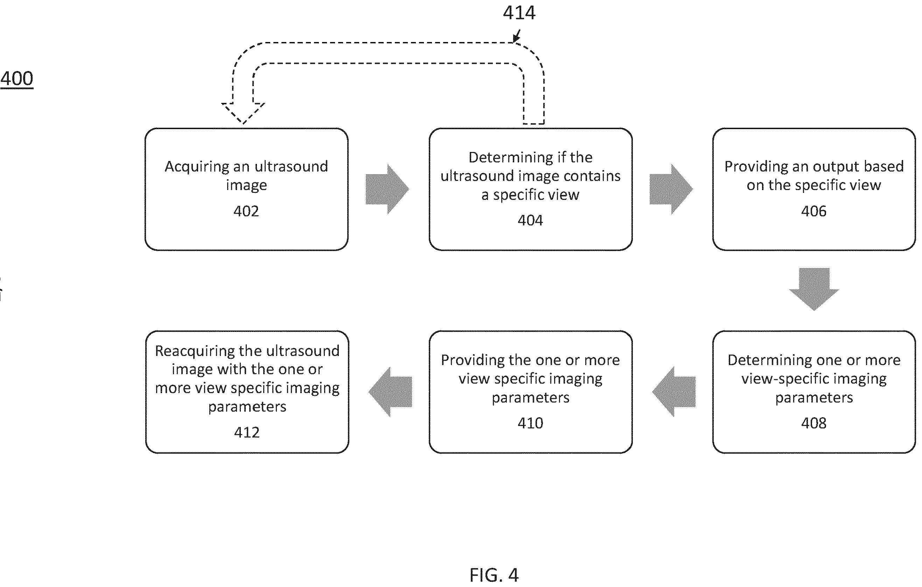

FIG. **4** is a flow diagram of a method 400 of ultrasound imaging performed in accordance with principles of the present disclosure. The processes at each of the blocks of method 400 may be performed in real time or live, that is, during real-time or live imaging of a subject. At block 402, a step of "acquiring an ultrasound image" may be performed. For example, the ultrasound image may be acquired by the ultrasound probe 112 of system 100, in some embodiments. The ultrasound image may be analyzed to determine whether it contains a specific view, as shown at block 404. This analysis and determination may be performed by a view recognition processor 170 according to any of the example herein. The view recognition processor 170 may include a neural network in some embodiments. In other embodiments, the view recognition processor 170 may use other image processing techniques to identify whether the specific view is represented in the acquired image. The processing of the ultrasound image at block 404 (e.g., by the view recognition processor 170) may further include determining a physiological state of the anatomy in the specific view and/or generating a confidence score of determination of the specific view. Upon determination that the ultrasound image corresponds to the specific view an output (e.g., a confirmation or indication of the specific view, a confidence score, etc.) may be provided, for example by the view recognition processor 170 to a downstream component of the system 100, as shown at block 406. The output may be a signal generated by the view recognition processor 170. If the ultrasound image does not correspond to the specific view, no output may be generated by the view recognition processor 170, or alternatively, a low (e.g., under 40%, or under 30%) may be output by the view recognition processor 170. In some embodiments, the method 400 may involve repeating blocks 402 and 404 , as indicated by dashed arrow 414, either until an output is generated at block 406 and/or until a confidence score of at least 50%, or in some cases at least 65% is output at block 404.

The method 400 may then proceed to block 408, at which a step of "determining one or more view-specific imaging parameters" may be performed. The determining may be performed by the optimization state controller 172 in some embodiments. The one or more imaging parameters may be based, at least in part, on the output (e.g., indication of specific view) from block 406. In some embodiments, absent an output (e.g., indication signal) from block 406, the ultrasound system may begin to or continue to generate images using default imaging parameters. The one or more default imaging parameters may be based on an exam type in some embodiments.

At block 410, a step of "providing the one or more view-specific imaging parameters," may be performed. The one or more imaging parameters, which may be the default imaging parameters, may be provided to a controller, such as the transmit controller 120 and/or beamformer 122 in some embodiments. In some embodiments, the optimization state controller 172 may wait for the indication to be provided for a period of time and/or an indication that the ultrasound probe is stationary before providing the one or more imaging parameters. At block 412, a step of "acquiring the ultrasound image with the one or more view-specific imaging parameters," may be performed. The acquiring may be performed by the ultrasound probe 112 under the control of the transmit controller 120 and/or beamformer 122.

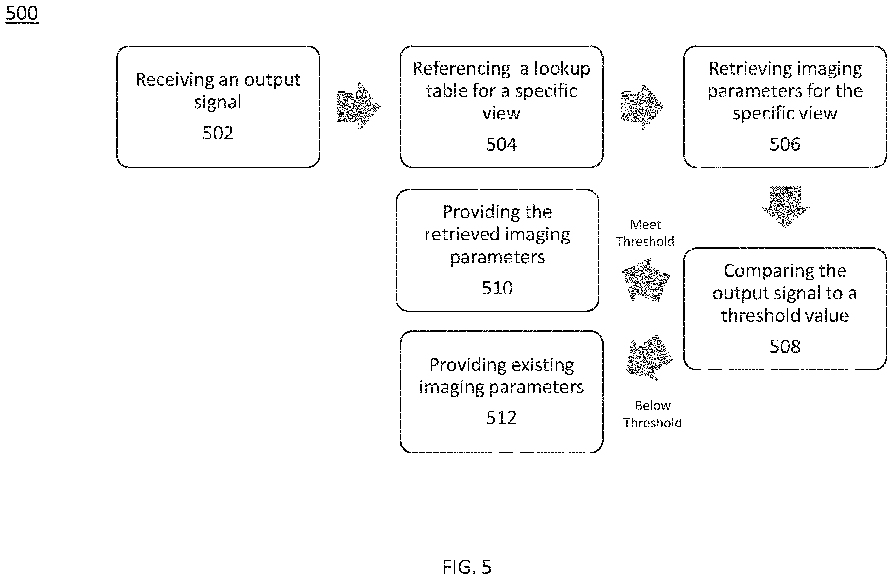

FIG. **5** is a flow diagram of a method 500 in accordance with principles of the present disclosure. In some embodiments, the method 500 may be performed by the optimization state controller 172. At block 502, a step of "receiving an output signal" may be performed. In some embodiments, the output signal may be provided by view recognition processor 170. The output signal may provide an indication of a specific view, a physiological state of anatomy in the specific view, and/or a confidence score in some embodiments. At block 504, a step of "referencing a lookup table for a specific view" may be performed. The specific view may be provided as the output signal or part of the output signal. At block 506, a step of "retrieving imaging parameters for the specific view" may be performed. The imaging parameters retrieved may be based on the lookup table. In some embodiments, the imaging parameters may be retrieved from local memory 142. In some embodiments, based on the specific view determined, one or more algorithms may be retrieved (e.g., from local memory). The one or more algorithms may be adaptive and may be used to provide different imaging parameters based, at least in part, on the specific view. For example, the one or more algorithms may provide different amounts of gain, different radio frequency (RF) filters, and/or image processing parameters to enhance the lateral wall of the heart when an apical 4-chamber view is detected.

At block 508, a step of "comparing the output signal to a threshold value" may be performed. In some embodiments, the threshold value may correspond to a threshold value of the confidence score. In some embodiments, the threshold value may be a number of ultrasound image frames or a time period for which the output signal remains stable, for example, the specific view indicated by the output signal remains constant. In some embodiments, the threshold value may correspond to a time period for which the ultrasound probe remains stationary. In some embodiments, the threshold value may be a combination of factors and/or multiple threshold values corresponding to different factors are analyzed (e.g., a confidence score above a threshold for a given number of frames). If the output signal meets or exceeds the threshold value or values, at block 510, a step of "providing the retrieved imaging parameters" may be performed. If the output signal is below the threshold, at block 512, a step of "providing existing imaging parameters" may be performed. Alternatively, at block 512, a step of "providing default imaging parameters" may be performed. In some embodiments, default parameters may be defined by the exam type (e.g., hepatic, fetal, cardiac). In some embodiments, block 512 may be performed in parallel with blocks 502, 504, 506, and/or 508 until the output signal meets or exceeds the threshold value.

In some embodiments, block 508 may be performed prior to blocks 504 and 506. In these embodiments, the output signal must meet or exceed the threshold value prior to blocks 504 and 506 being performed and block 510 may be performed after block 506. Further, in these embodiments, block 512 may be performed in parallel with 502 and/or 508.

The systems and methods described herein may allow for automatic adjustment of imaging parameters based on a specific view acquired by an ultrasound imaging system. This may allow for each specific view to be acquired with imaging parameters optimized for the specific view. Acquiring each view with optimized imaging parameters may improve the quality of images acquired without increasing the workload of a user.

Although the examples described herein refer to a current ultrasound exam or review of a prior exam, principles of the present disclosure can be applied to review of multiple exams. The exams may be of a single subject, for example, when reviewing a patient for the progression of a disease. The exams may be of multiple subjects, for example, when identifying an item of interest across a population for a medical study.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "C#", "Java", "Python", and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended scope of the claims that follow. Accordingly, the specification and drawings are to be regarded in an illustrative manner and are not intended to limit the scope of the appended claims.

## Claims

1. An ultrasound imaging system (100) comprising:
an ultrasound transducer array (114) configured to acquire an ultrasound image;
a controller (120) configured to control acquisition by the ultrasound transducer array based, at least in part, on one or more imaging parameters;
a view recognition processor (170) configured to determine if the ultrasound image corresponds to a specific view; and
an optimization state controller (172) configured to receive an output of the view recognition processor if the view recognition processor determines that the ultrasound image corresponds to the specific view, and to determine updates to the one or more imaging parameters, based, at least in part, on the output, wherein the optimization state controller provides the updated one or more imaging parameters to the controller; and
wherein the optimization state controller (172) is adapted to control the ultrasound imaging system (100) to reacquire an ultrasound image of the same specific view with the updated one or more imaging parameters if the ultrasound image has been stable for a certain period of time.

2. The ultrasound imaging system of claim 1, wherein the view recognition processor includes a neural network.

3. The ultrasound imaging system of claim 1, further comprising an image processor configured to process the ultrasound image based, at least in part, on the updated one or more imaging parameters determined by the optimization state controller.

4. The ultrasound imaging system of claim 1, wherein the view recognition processor is further configured to determine a physiological state of the specific view, wherein the physiological state is provided with the output.

5. The ultrasound imaging system of claim 4, wherein the physiological state is a phase of a cardiac cycle.

6. The ultrasound imaging system of claim 1, wherein the view recognition processor is further configured to determine a confidence score of the specific view, and wherein the optimization state controller is configured to provide the updated one or more imaging parameters to the controller when the confidence score is above a threshold value.

7. The ultrasound imaging system of claim 6, further comprising a user interface configured to receive a user input, wherein the user input includes the threshold value.

8. The ultrasound imaging system of claim 1, wherein the updated one or more imaging parameters varies across regions of the ultrasound image.

9. The ultrasound imaging system of claim 1, further comprising a memory, wherein the updated one or more imaging parameters are retrieved from the memory by the optimization state controller.

10. A method carried out on an ultrasound imaging system (100), the method comprising the steps:
acquiring an ultrasound image based on one or more imaging parameters;
determining if the ultrasound image contains a specific view;
if the specific view is determined, providing an output based on the specific view;
determining updates to the one or more imaging parameters, based at least in part, on the output;
providing the updated one or more imaging parameters to an optimization state controller (172) of the ultrasound imaging system (100); and
reacquiring an ultrasound image of the same specific view with the updated one or more imaging parameters if the ultrasound image has been stable for a certain period of time.

11. The method of claim 10, further comprising determining a physiological state of the specific view.

12. The method of claim 10, further comprising waiting for the output to be provided for a period of time prior to determining the one or more imaging parameters.

13. The method of claim 10, wherein if the output is not provided, determining one or more default imaging parameters and providing the one or more default parameters to the controller, and
optionally wherein, if the output is not provided, the method further comprises reacquiring the ultrasound image with the one or more default imaging parameters.

14. The method of claim 10, further comprising determining a confidence score of the specific view, wherein if the confidence score is above a threshold value, determining one or more imaging parameters, based at least in part, on the output, but if the confidence score is below the threshold value, determining one or more default imaging parameters and providing the one or more default imaging parameters to the controller.

15. A non-transitory computer-readable medium containing instructions, that when
executed, cause an ultrasound imaging system (100) to acquire an ultrasound image based on one or more imaging parameters; determine if the ultrasound image contains a specific view;
if the specific view is determined, provide an output based on the specific view; determine updates to the one or more imaging parameters, based at least in part, on the output;
provide the updated one or more imaging parameters to an optimization state controller (172) of the ultrasound imaging system (100); and
reacquire an ultrasound image of the same specific view, with the updated one or more imaging parameters if the ultrasound image has been stable for a certain period of time.

## Patentansprüche

1. Ultraschallbildgebungssystem (100), umfassend:
eine Ultraschallwandleranordnung (114), die konfiguriert ist, um ein Ultraschallbild zu erfassen;
eine Steuereinheit (120), die konfiguriert ist, um Erfassung durch die Ultraschallwandleranordnung zumindest teilweise basierend auf einem oder mehreren Bildgebungsparametern zu steuern;
einen Ansichtserkennungsprozessor (170), der konfiguriert ist, um zu bestimmen, ob das Ultraschallbild einer bestimmten Ansicht entspricht; und
eine Optimierungszustands-Steuereinheit (172), die konfiguriert ist, um eine Ausgabe des Ansichtserkennungsprozessors zu empfangen, wenn der Ansichtserkennungsprozessor bestimmt, dass das Ultraschallbild der bestimmten Ansicht entspricht, und um Aktualisierungen für den einen oder die mehreren Bildgebungsparameter zumindest teilweise basierend auf der Ausgabe zu bestimmen, wobei die Optimierungszustands-Steuereinheit der Steuereinheit den einen oder die mehreren aktualisierten Bildgebungsparameter bereitstellt; und
wobei die Optimierungszustands-Steuereinheit (172) angepasst ist, um das Ultraschallbildgebungssystem (100) zu steuern, um erneut ein Ultraschallbild derselben bestimmten Ansicht mit dem einen oder den mehreren aktualisierten Bildgebungsparametern zu erfassen, wenn das Ultraschallbild für einen bestimmten Zeitraum stabil war.

2. Ultraschallbildgebungssystem nach Anspruch 1, wobei der Ansichtserkennungsprozessor ein neuronales Netzwerk beinhaltet.

3. Ultraschallbildgebungssystem nach Anspruch 1, das weiter einen Bildprozessor umfasst, der konfiguriert ist, um das Ultraschallbild zumindest teilweise basierend auf dem einen oder den mehreren aktualisierten Bildgebungsparametern, die vom Optimierungszustands-Steuereinheit bestimmt wurden, zu verarbeiten.

4. Ultraschallbildgebungssystem nach Anspruch 1, wobei der Ansichtserkennungsprozessor weiter konfiguriert ist, um einen physiologischen Zustand der bestimmten Ansicht zu bestimmen, wobei der physiologische Zustand mit der Ausgabe bereitgestellt wird.

5. Ultraschallbildgebungssystem nach Anspruch 4, wobei der physiologische Zustand eine Phase eines Herzzyklus ist.

6. Ultraschallbildgebungssystem nach Anspruch 1, wobei der Ansichtserkennungsprozessor weiter konfiguriert ist, um einen Zuverlässigkeitswert der bestimmten Ansicht zu bestimmen, und wobei die Optimierungszustands-Steuereinheit konfiguriert ist, um der Steuereinheit den einen oder die mehreren aktualisierten Bildgebungsparameter bereitzustellen, wenn der Zuverlässigkeitswert über einem Schwellenwert liegt.

7. Ultraschallbildgebungssystem nach Anspruch 6, das weiter eine Benutzeroberfläche umfasst, die konfiguriert ist, um eine Benutzereingabe zu empfangen, wobei die Benutzereingabe den Schwellenwert beinhaltet.

8. Ultraschallbildgebungssystem nach Anspruch 1, wobei der eine oder die mehreren aktualisierten Bildgebungsparameter über Bereiche des Ultraschallbildes hinweg variieren.

9. Ultraschallbildgebungssystem nach Anspruch 1, das weiter einen Speicher umfasst, wobei der eine oder die mehreren aktualisierten Bildgebungsparameter durch die Optimierungszustands-Steuereinheit aus dem Speicher abgerufen werden.

10. Verfahren, das auf einem Ultraschallbildgebungssystem (100) durchgeführt wird, wobei das Verfahren die Schritte umfasst zum:
Erfassen eines Ultraschallbildes basierend auf einem oder mehreren Bildgebungsparametern;
Bestimmen, ob das Ultraschallbild eine bestimmte Ansicht enthält;
wenn die bestimmte Ansicht bestimmt ist, Bereitstellen einer Ausgabe basierend auf der bestimmten Ansicht;
Bestimmen von Aktualisierungen an dem einen oder den mehreren Bildgebungsparametern zumindest teilweise basierend auf der Ausgabe;
Bereitstellen des einen oder der mehreren aktualisierten Bildgebungsparameter für eine Optimierungszustands-Steuereinheit (172) des Ultraschallbildgebungssystems (100); und
erneutes Erfassen eines Ultraschallbildes derselben bestimmten Ansicht mit dem einen oder den mehreren aktualisierten Bildgebungsparametern, wenn das Ultraschallbild über einen bestimmten Zeitraum stabil war.

11. Verfahren nach Anspruch 10, das weiter Bestimmen eines physiologischen Zustands der bestimmten Ansicht umfasst.

12. Verfahren nach Anspruch 10, das weiter Warten darauf umfasst, dass die Ausgabe für einen Zeitraum vor dem Bestimmen des einen oder der mehreren Bildgebungsparameter bereitgestellt wird.

13. Verfahren nach Anspruch 10, wobei, wenn die Ausgabe nicht bereitgestellt wird, Bestimmen eines oder mehrerer Standard-Bildgebungsparameter und Bereitstellen des einen oder der mehreren Standardparameter für die Steuereinheit, und
wobei optional, wenn die Ausgabe nicht bereitgestellt wird, das Verfahren weiter erneutes Erfassen des Ultraschallbildes mit dem einem oder den mehreren Standard-Bildgebungsparametern umfasst.

14. Verfahren nach Anspruch 10, das weiter Bestimmen eines Zuverlässigkeitswerts der bestimmten Ansicht umfasst, wobei, wenn der Zuverlässigkeitswert über einem Schwellenwert liegt, ein oder mehrere Bildgebungsparameter zumindest teilweise basierend auf der Ausgabe bestimmt werden, wenn der Zuverlässigkeitswert jedoch unter dem Schwellenwert liegt, ein oder mehrere Standardbildgebungsparameter bestimmt werden und der Steuereinheit der eine oder die mehreren Standardbildgebungsparameter bereitgestellt werden.

15. Nichtflüchtiges computerlesbares Medium, das Anweisungen enthält, die, wenn sie ausgeführt werden, ein Ultraschallbildgebungssystem (100) veranlassen:
ein Ultraschallbild basierend auf einem oder mehreren Bildgebungsparametern zu erfassen;
zu bestimmen, ob das Ultraschallbild eine bestimmte Ansicht enthält;
wenn die bestimmte Ansicht bestimmt ist, eine Ausgabe basierend auf der bestimmten Ansicht bereitzustellen;
Aktualisierungen für den einen oder die mehreren Bildgebungsparameter zumindest teilweise basierend auf der Ausgabe zu bestimmen;
einer Optimierungszustands-Steuereinheit (172) des Ultraschallbildgebungssystems (100) den einen oder die mehreren aktualisierten Bildgebungsparameter bereitzustellen; und
erneut ein Ultraschallbild derselben bestimmten Ansicht, mit dem einen oder den mehreren aktualisierten Bildgebungsparametern zu erfassen, wenn das Ultraschallbild über einen bestimmten Zeitraum stabil war.

## Revendications

1. Système d'imagerie ultrasonore (100) comprenant :
un réseau de transducteurs à ultrasons (114) configuré pour acquérir une image ultrasonore ;
un dispositif de commande (120) configuré pour commander l'acquisition par le réseau de transducteurs à ultrasons sur la base, au moins en partie, d'un ou plusieurs paramètres d'imagerie ;
un processeur de reconnaissance de vue (170) configuré pour déterminer si l'image ultrasonore correspond à une vue spécifique ; et
un dispositif de commande d'état d'optimisation (172) configuré pour recevoir une sortie du processeur de reconnaissance de vue si le processeur de reconnaissance de vue détermine que l'image ultrasonore correspond à la vue spécifique, et pour déterminer des mises à jour des un ou plusieurs paramètres d'imagerie, sur la base, au moins en partie, de la sortie, dans lequel le dispositif de commande d'état d'optimisation fournit les un ou plusieurs paramètres d'imagerie mis à jour au dispositif de commande ; et
dans lequel le dispositif de commande d'état d'optimisation (172) est conçu pour commander le système d'imagerie ultrasonore (100) pour réacquérir une image ultrasonore de la même vue spécifique avec les un ou plusieurs paramètres d'imagerie mis à jour si l'image ultrasonore est stable depuis un certain laps de temps.

2. Système d'imagerie ultrasonore selon la revendication 1, dans lequel le processeur de reconnaissance de vue inclut un réseau neuronal.

3. Système d'imagerie ultrasonore selon la revendication 1, comprenant en outre un processeur d'image configuré pour traiter l'image ultrasonore sur la base, au moins en partie, des un ou plusieurs paramètres d'imagerie mis à jour déterminés par le dispositif de commande d'état d'optimisation.

4. Système d'imagerie ultrasonore selon la revendication 1, dans lequel le processeur de reconnaissance de vue est en outre configuré pour déterminer un état physiologique de la vue spécifique, dans lequel l'état physiologique est fourni avec la sortie.

5. Système d'imagerie ultrasonore selon la revendication 4, dans lequel l'état physiologique est une phase d'un cycle cardiaque.

6. Système d'imagerie ultrasonore selon la revendication 1, dans lequel le processeur de reconnaissance de vue est en outre configuré pour déterminer un score de confiance de la vue spécifique, et dans lequel le dispositif de commande d'état d'optimisation est configuré pour fournir les un ou plusieurs paramètres d'imagerie mis à jour au dispositif de commande lorsque le score de confiance est supérieur à une valeur seuil.

7. Système d'imagerie ultrasonore selon la revendication 6, comprenant en outre une interface utilisateur configurée pour recevoir une entrée utilisateur, dans lequel l'entrée utilisateur inclut la valeur seuil.

8. Système d'imagerie ultrasonore selon la revendication 1, dans lequel les un ou plusieurs paramètres d'imagerie mis à jour varient selon les régions de l'image ultrasonore.

9. Système d'imagerie ultrasonore selon la revendication 1, comprenant en outre une mémoire, dans lequel les un ou plusieurs paramètres d'imagerie mis à jour sont extraits de la mémoire par le dispositif de commande d'état d'optimisation.

10. Procédé mis en œuvre sur un système d'imagerie ultrasonore (100), le procédé comprenant les étapes suivantes :
l'acquisition d'une image ultrasonore sur la base d'un ou plusieurs paramètres d'imagerie ;
la détermination pour établir si l'image ultrasonore contient une vue spécifique ;
si la vue spécifique est déterminée, la fourniture d'une sortie sur la base de la vue spécifique ;
la détermination de mises à jour des un ou de plusieurs paramètres d'imagerie, sur la base, au moins en partie, de la sortie ;
la fourniture des un ou plusieurs paramètres d'imagerie mis à jour à un dispositif de commande d'état d'optimisation (172) du système d'imagerie ultrasonore (100) ; et
la réacquisition d'une image ultrasonore de la même vue spécifique avec les un ou plusieurs paramètres d'imagerie mis à jour si l'image ultrasonore est stable depuis un certain laps de temps.

11. Procédé selon la revendication 10, comprenant en outre la détermination d'un état physiologique de la vue spécifique.

12. Procédé selon la revendication 10, comprenant en outre l'attente de la fourniture de la sortie pendant un laps de temps avant de déterminer les un ou plusieurs paramètres d'imagerie.

13. Procédé selon la revendication 10, dans lequel, si la sortie n'est pas fournie, la détermination d'un ou plusieurs paramètres d'imagerie par défaut et la fourniture des un ou plusieurs paramètres par défaut au dispositif de commande, et
optionnellement dans lequel, si la sortie n'est pas fournie, le procédé comprend en outre la réacquisition de l'image ultrasonore avec les un ou plusieurs paramètres d'imagerie par défaut.

14. Procédé selon la revendication 10, comprenant en outre la détermination d'un score de confiance de la vue spécifique, dans lequel si le score de confiance est supérieur à une valeur seuil, la détermination d'un ou plusieurs paramètres d'imagerie, sur la base, au moins en partie, de la sortie, mais si le score de confiance est inférieur à la valeur seuil, la détermination d'un ou plusieurs paramètres d'imagerie par défaut et la fourniture des un ou plusieurs paramètres d'imagerie par défaut au dispositif de commande.

15. Support non transitoire lisible par ordinateur contenant des instructions qui, lorsqu'elles sont exécutées, amènent un système d'imagerie ultrasonore (100) à :
acquérir une image ultrasonore sur la base d'un ou plusieurs paramètres d'imagerie ;
déterminer si l'image ultrasonore contient une vue spécifique ;
si la vue spécifique est déterminée, fournir une sortie sur la base de la vue spécifique ;
déterminer des mises à jour des un ou de plusieurs paramètres d'imagerie, sur la base, au moins en partie, de la sortie ;
fournir les un ou plusieurs paramètres d'imagerie mis à jour à un dispositif de commande d'état d'optimisation (172) du système d'imagerie ultrasonore (100) ; et
réacquérir une image ultrasonore de la même vue spécifique, avec les un ou plusieurs paramètres d'imagerie mis à jour si l'image ultrasonore est stable depuis un certain laps de temps.
